Europäisches Patentamt

European Patent Office

Office européen des brevets

(19)

(11) **EP 1 632 240 A1**

(12) **EUROPEAN PATENT APPLICATION**

(43) Date of publication:
**08.03.2006 Bulletin 2006/10**

(51) Int Cl.:
***A61K 36/57*** *(2006.01)*     ***A61K 36/575*** *(2006.01)*

(21) Application number: **05254158.8**

(22) Date of filing: **01.07.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **01.07.2004 US 880651**

(71) Applicant: **Yangsen Biotechnology Co., Ltd Pei-Tou Taipei (TW)**

(72) Inventors:
 • **Lin, Chun-Ying Tapiei 112 (TW)**

 • **Hsu, Chih-Chieh Tapiei 112 (TW)**
 • **Hsu, Hui-Chun Tapiei 112 (TW)**
 • **Yang, Wen-Jai Tapiei 112 (TW)**
 • **Tsai, Ying-Chieh Tapiei 112 (TW)**

(74) Representative: **Wright, Simon Mark J.A. Kemp & Co. 14 South Square Gray's Inn London WC1R 5JJ (GB)**

(54) **Essential oils for treating and/or preventing allergic disease**

(57)     The present invention provides the essential oil and the essential oil such as Magnolia biondii, Agastache rugosa, and Notopterygium forbesii, and combinations thereof composition of traditional Chinese herbal medicine to prevent and/or treat allergic disease. The essential oil and the essential oil composition can be formulated as a solution, a cream, a lotion, ointment, or a solid stick, and applied topically.

EP 1 632 240 A1

## Description

### Field of the Invention

The present invention relates to the novel use and compositions of herbal medicine for treating and/or preventing an allergic disease (or condition) or allergies (such as allergic rhinitis and/or chronic rhinitis).

### Background of the Invention

Allergy refers to any condition of the body in which an attack is mounted on a specific foreign substance. People can experience allergic reactions to foods, chemicals, plants, animals and a variety of airborne substances. The substance to which the person is allergic is called the allergen. Allergy is defined as a hypersensitivity or hyperimmunity caused by exposure to a particular antigen (allergen) resulting in a marked increase in reactivity to that antigen/allergen upon subsequent exposure. The symptoms of allergic diseases, such as allergic rhinitis (hay fever), allergic dermatitis, and allergic asthma, can be caused by a variety of atopic allergens, such as grasses, trees, weeds, animal dander, insects, moulds, drugs, and chemicals. For example, allergic rhinitis is a very common allergic disease worldwide and is a kind of type 1 hypersensitivity. Allergic rhinitis refers to an inflammatory condition of nose cause by an allergy and affects millions of people throughout the world. The typical symptoms of allergic rhinitis include sneezing, itchy nose, nasal congestion and rhinorrhea ("running" or "runny" nose ), often accompanied by watery and itchy eyes, and postnasal drip. These allergic diseases are mediated by an antibody known as immunoglobulin E, or simply IgE. Anti-IgE medicines that reduce IgE levels are attractive treatments for allergy patients.

IgE is a member of the immunoglobulin family that mediates allergic responses such as asthma, food allergies, type 1 hypersensitivity, and the familiar sinus inflammation suffered on a widespread basis. IgE bonds to mast cells and basophiles. Upon combination of a specific allergen with IgE bound to mast cells or basophiles, the IgE may be cross-linked on the cell surface, resulting in the physiological effects of IgE-antigen interaction. This may result in the release of histamine, serotonin, heparin, a chemotactic factor for eosinophilic leukocytes and/or the leukotrienes, C4, D4, and E4, which cause prolonged constriction of bronchial smooth muscle cells. These released substances are the mediators, which result in allergic symptoms.

Immunosuppressant compounds induce an inhibition of the immune response system and are customarily used in the treatment of allergic disease. Compounds which are known to exhibit immunosuppressant activity include the fungal metabolite Cyclosporin A and the macrolide antibiotic (a metabolite from Streptomyces tsukabaensis) termed FK506. Both of these agents have been used clinically and experimentally to suppress the immune system in transplantation and in the treatment of a number of diseases. As for allergic rhinitis, some pharmaceuticals, such as antihistamines, decongestants, corticosteroids and mast cell stabilizers, are used for treating and preventing allergic rhinitis. However, they are not necessarily satisfactory, because they are not entirely effective against the nasal congestion of allergic rhinitis and have side effects such as sleepiness, malaise, and sedation due to inhibitory action on the central nervous system (Ju Lih-Ling, The Journal of Pharmacy, Volume 58, page 79-86, 1999). In contrast, NSAIDS are also very useful as anti-inflammatory drugs. In addition, NSAIDS and anti-inflammatory steroid agents have a cytokine-production suppressing effect and have been used for autoimmune diseases.

Pharmaceutical agents for allergic diseases have been developed and used for therapy. However, since such pharmaceutical agents have adverse effects, there has been a strong demand for anti-allergic agents derived from natural products, where long-term administration is possible, safety is high, and no adverse reaction takes place.

### Summary Of the Invention

A first aspect of the invention is to provide a composition, suitable for use in the treatment and/or prevention of allergic disease (or allergy), or allergic rhinitis and/or chronic rhinitis, which comprises an oil (derived) from certain plants, such as an essential oil, e.g. the essential oil of HOUXIANG HERBA, the (e.g. essential) oil of QIANGHUO HERBA and/or the (e.g. essential) oil of XINYI HERB, wherein HOUXIANG HERBA can comprise a plant (selected from the group consisting of) *Agastache rugosa, Pogostemon cablin, Pogostemon formosanum, Microtoena delavayi, Microtoena insuavis, Microtoena patchouli, Teucrium quadrifarium, Epimeredi indica* and *Anisomeles indica;* QIANGHUO HERBA can comprise a plant (selected from the group consisting of) *Notopterygium forbesii* and *Notopterygium incisum;* and XINYI HERB can comprise a plant (selected from the group consisting of) *Magnolia biondii, Magnolia denudata Dear., Magnolia denudata Desr., Magnolia pilocarpa, Magnolia liliiflora, Magnolia sargentiana, Magnolia cempbelli Hook., Magnolia martini Dandy,* and *Magnolia sprengeri.*

Another object of the invention is to provide the use of an oil (such as of the first aspect) for the manufacture of a composition (e.g. medicament) for treating and/or preventing allergic disease or allergy, the composition comprising an oil (derived) from a plant such as an essential oil of HOUXIANG HERBA, wherein HOUXIANG HERBA comprises a plant selected from the group consisting of *Agastache rugosa, Pogostemon cablin, Pogostemon formosanum*, *Microtoena delavayi*, *Microtoena insuavis*, *Microtoena patchouli*, *Teucrium quadrifariurn*, *Epimeredi indica* and *Anisomeles indica.* Preferably, the (essential) oil is (obtained) from *Agastache rugosa.*

A further object of the present invention is to provide a composition, for treating and/or preventing allergic disease, comprising an effective and sufficient amount of (essential) oil the first aspect, such as of QIANGHUO HERBA (RHIZOMA ET RADIX NOTOPTERYGH) to a patient for treating and/or preventing allergic disease, wherein QIANGHUO HERBA comprises the plants selected from the group consisting of *Notopterygium forbesii* and *Notopterygium incisum.*

## Brief Description of the Drawings

Figure 1 shows the average number of nasal rubbings for each test group.
Figure 2 shows the average number of sneezes for each test group.

## Detailed Description of the Invention

The present invention provides compositions for use in methods for the treatment of allergic disease or allergy by using a plant (e.g. essential) oil(s) and the pharmaceutical composition comprising the plant (e.g. essential) oil(s). According to the invention, the term "essential oil" can refer to the (e.g. highly scented) droplets found in minute quantities in and so can be derived, or isolated from, the flowers, stems, leaves, roots and/or barks of aromatic plants. They may not be true oils in the manner of lubricant vegetable oils, but (highly) fluid and (e.g. exceptionally) volatile. Essential oils can be complex mixtures (or comprise) different organic molecules -- terpenes, alcohols, esters, aldehydes, ketones, and/or phenols. In view of the volatility and pleasant aroma, plant essential oils can have advantageous benefits such as their reduction of subjects' complaints about uncomfortable administration and their ease of use.

## Compositions of Herbal Essential Oils

One aspect of the invention is to provide a composition, for use in the treatment and/or prevention of allergic disease (or allergy), which comprises the (essential) oil of HOUXIANG HERBA and/or the (essential) oil of XINYI HERB (or, instead, magnosalin), wherein HOUXIANG HERBA comprises a plant selected from the group consisting of *Agastache rugosa, Pogostemon cablin, Pogostemon formosanum, Microtoena delavayi, Microtoena insuavis, Microtoena patchouli, Teucrium quadrifarium, Epimeredi indica* and *Anisomeles indica* and XINYI HERB comprises a plant selected from the group consisting of *Magnolia biondii, Magnolia denudata Dear., Magnolia denudata Desr., Magnolia pilocarpa, Magnolia liliiflora, Magnolia sargentiana, Magnolia cempbelli Hook., Magnolia martini Dandy,* and *Magnolia sprengeri.* Preferably, HOUXIANG HERBA is *Agastache rugosa* and XINYI HERBA is *Magnolia biondii.* According to one embodiment of the invention, the (e.g. essential) oil of HOUXIANG HERB in the composition can range from 0.05 to 0.95% (or parts) by volume, and/or the (e.g. essential) oil of XINYI HERB in the composition ranges from 0.05 to 0.95% (or parts) by volume.

Another aspect of the invention is to provide a composition for use in the treatment and/or prevention of allergic disease (or allergy), which comprises the (e.g. essential) oil of QIANGHUO HERBA and/or the (e.g. essential) oil of XINYI HERB, wherein QIANGHUO HERBA comprises a plant (selected from the group consisting of) *Notopterygium forbesii* and *Notopterygium incisum* and XINYI HERB comprises a plant (selected from the group consisting of) *Magnolia biondii*, *Magnolia denudata Dear.*, *Magnolia denudata Desr.*, *Magnolia pilocarpa, Magnolia liliiflora, Magnolia sargentiana, Magnolia cempbelli Hook., Magnolia martini Dandy,* and *Magnolia sprengeri.* Preferably, QIANGHUO HERBA is *Notopterygium forbesii* and/or XINYI HERB is *Magnolia biondii.* According to one embodiment of the invention, the (essential) oil of QIANGHUO HERB in the composition can range from 0.05 to 0.95% (or parts) by volume, and/or the (essential) oil of XINYI HERB in the composition can range from 0.05 to 0.95% (or parts) by volume. Preferably the concentrations of the (or each) oil in the composition is from 0.05 to 20, such as 0.1 to 10, optimally from 2 to 5, μg/ml. If 2 (or 3) oils are included preferably they are present in approximately equal amounts (e.g. a ration of about 1:1, or 1:1:1).

Another aspect of the invention is to provide a composition, for use in the treatment and/or prevention of allergic disease (or allergy), which comprises the (essential) oil of HOUXIANG HERBA, the (essential oil) of QIANGHUO HERB and/or the (essential) oil of XINYI HERB, wherein HOUXIANG HERB comprises a plant (selected from the group consisting of) *Agastache rugosa, Pogostemon cablin, Pogostemon formosanum, Microtoena delavayi, Microtoena insuavis, Microtoena patchouli, Teucrium quadrifarium, Epimeredi indica* and *Anisomeles indica;* QIANGHUO HERBA comprises a plant (selected from the group consisting of) *Notopterygium forbesii* and *Notopterygium incisum;* and XINYI HERB comprises a plant (selected from the group consisting of) *Magnolia biondii, Magnolia denudata Dear., Magnolia denudata Desr., Magnolia pilocarpa, Magnolia liliiflora, Magnolia sargentiana, Magnolia cempbelli Hook., Magnolia martini Dandy,* and *Magnolia sprengeri.* Preferably, HOUXIANG HERBA is *Agastache rugosa,* QIANGHUO HERBA is *Notopterygium forbesii* and XINYI HERB is *Magnolia biondii.* The composition can comprise 0.5% to 99.0% by volume of the (essential) oil of HOUXIANG HERBA, 0.5% to 99.0% by volume of the (essential) oil of QIANGHUO HERBA, and 0.5% to 99.0% by volume of the (essential) oil of XINYI HERB. Preferably, the composition can comprise 0.5% to 49.5% by volume of the (essential) oil of HOUXIANG HERBA, 0.5% to 49.5% by volume of the (essential) oil of QIANGHUO HERBA, and/or 0.5 to 49.5% (or the remaining 50.0% to 99.0%) by volume of the (essential) oil of XINYI HERB.

HOUXIANG HERBA is a known Chinese herbal medicine and used for treating gastrointestinal diseases such as abdominal pain, gastritis, enteritis, and gastrotympanities. According to the invention, HOUXIANG HERBA refers to a plant (selected from the group consisting of) *Agastache rugosa, Pogostemon cablin, Pogostemon formosanum, Microtoena delavayi, Microtoena insuavis, Microtoena patchouli, Teucrium quadrifarium, Epimeredi indica* and *Anisomeles indica.* It is known that the plants of HOUXIANG HERBA have similar pharmaceutical effects in Chinese medicine (Zhou De-Zhen, Master's Thesis, Graduate School of Industry and Commerce, Chinese Culture University, 1970). Therefore, the plants *Agastache rugosa, Pogostemon cablin, Pogostemon formosanum, Microtoena delavayi, Microtoena insuavis, Microtoena patchouli, Teucrium quadrifarium, Epimeredi indica* and *Anisomeles indica* are mutually replaceable when used as pharmaceuticals.

XINYI HERB is a kind of traditional Chinese medicine and belongs to the Magnoliaceae family. According to the invention, XINYI HERB refers to a plant (selected from the group consisting of) *Magnolia biondii, Magnolia denudata Dear., Magnolia denudata Desr., Magnolia pilocarpa, Magnolia liliiflora, Magnolia sargentiana, Magnolia cempbelli Hook., Magnolia martini Dandy,* and *Magnolia sprengeri.* The herb is usually collected in early spring when the bud is not in blossom, dried in the sun, and cleared of stalks. It is known that the herb is often used for the treatment of chronic rhinitis, allergic rhinitis, and maxillary sinusitis. Kobayashi et al. indicated that magnosalin, a compound derived from *Fols magnoliae,* has inhibitory effects on the proliferation of synovial cells in rheumatoid arthritis models (Kobayashi et al., Immunopharmacology 39(2): 139-147, 1998 May), and this can be used to replace XINYI HERB. The herb is traditionally taken orally. Furthermore, Wang (W.K. Wang et al., Journal of Shaanxi College of Tradition Chinese Medicine, 2000, Vol. 23., No. 2, pg. 40-42) has described Flos magnoliae as inhibiting allergies when administered orally.

QIANGHUO HERBA (RHIZOMA ET RADIX NOTOPTERYGH) is a traditional Chinese medicine and used in treating osteonecrosis, gout, arthritis, brain diseases, wounds, mixed neoplasms, digestive system neoplasms, and connective and soft tissue neoplasms (CN 99108294 and CN 98122088). According to the invention, QIANGHUO HERBA refers to a plant selected from the group consisting of *Notopterygium forbesii* and *Notopterygium incisum. Notopterygium forbesii* and *Notopterygium incisum* belong to the *Notopterygium* family and have similar pharmaceutical effects.

According to the invention, the composition may further comprise one or more (e.g. essential) oils, such as eucalyptus oil, lemon oil, menthol oil, almond oil, jojoba oil, rosehip oil, sesame oil, coconut oil, evening primrose oil, anise oil, cinnamon oil, lavender oil, bergamot oil, and/or avocado oil. Preferably, the other (essential) oil is (selected from) eucalyptus oil, lemon oil, menthol oil, and/or bergamot oil. In addition, the composition can further comprise another agent for treating allergic rhinitis or chronic rhinitis known to persons skilled in the art, such as menthol oil, α-adrenergic decongestionants, (pseudoephedrine, phenylpropanolamine, oximethazoline, tramazoline), adrenergic bronchodilatators (epinephrine, isoproterenol, salbutamol, terbutaline, pirbuterol and metaproterenol), antihistamines (azelastine, levocabastine), corticosteroids (beclomethasone dipropionate, flunisolide), or the more preferable sodium chromoglycate and ipatropium bromide.

New Use of Essential Oils of HOUXIANG HERBA and QIANGHUO HERBA

Another aspect of the invention is to provide a composition for use in methods of treating and/or preventing allergic disease (or allergic rhinitis or chronic rhinitis), the composition comprising administering an effective and sufficient amount of essential oil of HOUXIANG HERBA, wherein HOUXIANG HERBA comprises the plants selected from the group consisting of *Agastache rugosa, Pogostemon cablin, Pogostemon formosanum, Microtoena delavayi, Microtoena insuavis, Microtoena patchouli, Teucrium quadrifarium, Epimeredi indica* and *Anisomeles indica.* Preferably, the essential oil is obtained from *Agastache rugosa.*

A further aspect of the invention is to provide a composition for use in methods of treating and/or preventing allergic disease (or allergic rhinitis or chronic rhinitis), comprising administering an effective and sufficient amount of essential oil of QIANGHUO HERBA, wherein QIANGHUO HERBA comprises the plants selected from the group consisting of *Notopterygium forbesii* and *Notopterygium incisum.* Preferably, the essential oil is obtained from *Notopterygium forbesii.*

Preparation and Formulation of Herbal Essential Oils

According to the invention, the essential oils of the above-mentioned herbs can be prepared by extraction methods known in the art. Preferably, the (essential) oil of the plants is extracted by water distillation.

According to the invention, the essential oil, or the essential oil composition, can be formulated as a solution, a cream, a lotion, an ointment, or a solid stick. The composition or formulation can be directly applied to the topical skin of whole body, and the transdermal essential oil or the essential oil composition can infiltrate the skin to take effect. Especially when applying to the area between the nose and upper lip, the essential oil or the essential oil composition volatilizes and then can reach or be administered to the nasal and respiratory mucosa by inhalation.

For the solution formulation, the essential oil or the essential oil composition is optionally diluted with organic solvents that are non-toxic to humans, such as menthol, ethanol and isopropanol. The (solution) formulation can also be delivered by any one of a variety of inhalation devices. The term "inhalation device" refers to a device used to administer an essential oil/or other volatile oils as well known to persons skilled in the art, such as liquid nebulizers, sprayers, thermal vaporizers, burners, aromatherapy lamps, ultrasonic vaporizers, steamers, electrohydrodynamic aerosolizers, etc. In addition, the inhalation device must be practical, in the sense of being easy to use and durable. Preferably, the essential oil or the essential oil composition is delivered by using an aromatherapy lamp.

For the cream, ointment, or lotion formulation, the essential oil or the essential composition can be formulated in a suitable carrier. To prepare the essential oil or the essential composition in lotion, cream or ointment form, the essential oil or the essential composition can be first dissolved in a suitable carrier such as water, ethanol, propylene glycol, and/or another vehicle. The solution thus obtained can then be mixed with a desired base to make lotion, cream or ointment.

For the stick formulation, the essential oil or the essential oil composition can be combined with appropriate carriers to form a solid stick. The methods for preparing the stick are known in the art. Preferably, the carrier is menthol. The stick formulation can be directly applied to the topical skin of the whole body and the transdermal essential oil or the essential oil composition can infiltrate the skin to take effect. Especially when applying it to the area between the nose and upper lip, the essential oil or the essential oil composition volatilizes and then can reach or be administered to the nasal and respiratory mucosa by inhalation.

According to the invention, the essential oil or the essential oil composition can be administered by inhalation, percutaneous absorption or oral administration. Preferably, the essential oil or the essential oil composition is delivered by inhalation to achieve a reduced side effect and a fast uptake by the nasal and respiratory mucosa to relieve the symptoms of the allergic rhinitis or chronic rhinitis. The quantity of the essential oil or the essential oil composition administrated, and the duration of administration of a single dose all depend on the type of inhalation device employed and the formulation, as those skilled in the art would recognize.

For inhalation the essential oil can be administered by using a liquid nebulizer, a sprayer, thermal vaporizer, a burner, aromatherapy lamp, an ultrasonic vaporizer, a steamer, or an electrohydrodynamic aerosolizer. Normally, the amount of the essential oil or the essential oil composition ranges from about 10% to 100% by volume. Preferably, using an aromatherapy lamp, the formulations of the essential oil or the essential oil composition typically include the essential oil in a solution at an amount of about 10% to 15% by volume.

For percutaneous absorption, the essential oil or the essential oil composition can be an essential oil patch, mask, cream, or lotion. The essential oil can penetrate the skin and achieve a therapeutic effect.

For oral therapeutic administration, the essential oil may be diluted in water and/or potatory liquid.

According to the present invention, the dosage (e.g. of agent) oil or composition that can be amount administered to a patient can range from about 0.01 ml/day to 0.05ml/day. Preferably, the dosage administered to a patient is about 0.02ml/per day. Administration of the essential oil can include chronic, acute, or intermittent regimens, and any mode where essential oil is feasible may be selected.

According to the invention, the essential oil or the essential oil composition can be used in the treatment and/or prevention of allergic disease. When a mammal is exposed to an allergen, the immune system will be induced to produce the specific antibody IgE. The allergen binds the IgE to form an Ag-Ab (allergen-antibody) complex, so that macrophages and mast cells release NO, histamine, platelet-activating factor (PAF) and other factors. According to the invention, the allergic diseases (or allergy) can include allergic rhinitis, allergic pneumonia, allergic conjunctivitis, allergic dermatitis, allergic sinusitis, allergic asthma, allergic bronchitis, allergic arthritis, and allergic arteritis and food allergies. Preferably, the allergic disease is allergic rhinitis.

**EXAMPLES**

Example 1: Inhibitory effect of essential oils on NO production by LPS-activated mouse macrophage cell line RAW 246.7

**A) Cell culture**

The mouse macrophage cell line RAW 264.7 was purchased from Bioresources Collection & Research Center, Taiwan and numbered CCRC 60001. The cells were added to Dulbecco's Modified Eagle Medium (DMEM, GibcoBRL) containing 10%(v/v) fetal bovine serum (FBS, GibcoBRL) and then incubated in a $CO_2$ incubator containing 5% $CO_2$ at 37°C. The cells grew to 90% density and were then washed with phosphate buffered saline (PBS). The cells were colleted and diluted with fresh DMEM to the appropriate concentration.

**B) Determination of Production of Nitric Oxide**

The mouse macrophage cells were transferred in a 96-well plate in a concentration of 10,000 cells/well. After incubating for 24 hours, the cells were activated with the medium containing 100 μg/ml Lipopolysaccharide (LPS, SIGMA) and 100 μg/ml Interferon-γ(INF-γ, GibcoBRL). Meanwhile, the essential oils of herbal medicine in different concentrations were added to the wells. After incubating for 24 hours, 50 μl of supernatants were respectively taken and put into a 96-well plate. 50μl Griess reagent A (60mM sulfanilamide solved in 3 N HCL) and 50 μl Griess reagent B (4mM naphthylethylene diamine dihydrochloride) were respectively added to the cell supernatants and reacted at room temperature for 10 minutes. Then, the absorbance of the resulting cell supernatants was determined with a microplate reader at a wavelength of 500mm. The percentage of nitric oxide can be calculated by the following formula:

$$\% \text{ nitric oxide} = (\text{sample absorptivity} / \text{control absorptivity}) \times 100\%.$$

The results are shown in below Table 1.

Table 1

| Samples | Concentration(μg/ml) | Nitric oxide(μM) |
|---|---|---|
| Blank | | 3.5±0.3 |
| LPS (Inducer) | | 60±0.5 |
| L-NMMA (positive control) | | 25±0.6 |
| *Magnolia biondii* | 0.1 μg/ml | 40.8±0.4 |
| *Magnolia biondii* | 1μg/ml | 37.8±0.2 |
| *Magnolia biondii* | 10μg/ml | 33.1±0.3 |
| *Agastache rugosa* | 0.1 μg/ml | 40.8±0.5 |
| *Agastache rugosa* | 1 μg/ml | 33.6±0.4 |
| *Agastache rugosa* | 10μg/ml | 30.2±0.3 |
| *Notopterygium forbesii* | 0.1 μg/ml | 49.7±0.2 |
| *Notopterygium forbesii* | 1 μg/ml | 45.5±0.3 |
| *Notopterygium forbesii* | 10μg/ml | 43.1±0.2 |
| *Magnolia biondii + Agastache rugosa* (1:1 by volume) | 0.1 μg/ml | 35±0.3 |
| *Magnolia biondii + Agastache rugosa* (1:1 by volume) | 1μg/ml | 30±0.2 |
| *Magnolia biondii + Agastache rugosa* (1:1 by volume) | 10μg/ml | 27±0.1 |
| *Magnolia biondii + Notopterygium forbesii* (1:1 by volume) | 0.1μg/ml | 38±0.4 |
| *Magnolia biondii + Notopterygium forbesii* (1:1 by volume) | 1μg/ml | 35±0.3 |
| *Magnolia biondii + Notopterygium forbesii* (1:1 by volume) | 10μg/ml | 32±0.1 |
| *Magnolia biondii + Agastache rugosa + Notopterygium forbesii* (1:1:1 by volume) | 0.1μg/ml | 34±0.2 |
| *Magnolia biondii + Agastache rugosa + Notopterygium forbesii* (1:1:1 by volume) | 1μg/ml | 28±0.3 |
| *Magnolia biondii + Agastache rugosa + Notopterygium forbesii* ( 1:1:1 by volume) | 10μg/ml | 24±0.2 |

Example 2: Inhibitory Activity of Essential Oils on PAF-induced Platelet Aggregation

**A) Preparation of platelet suspension**

The anticoagulant "acid-citrate-dextrose" (ACD) was added to the blood taken from a New Zealand rabbit in a ratio of 1:6 by volume. The resulting blood was centrifuged at 190g for 15 min. at room temperature. After centrifugation, the supernatant containing platelet-rich plasma (PRP) was taken and 0.2 M EDTA was added. The resulting supernatant was centrifuged at 1000g for 1.5 min. After centrifugation, the pellet containing RBC and WBC was removed and then PRP was centrifuged at 1000g for 11 min. After centrifugation, the supernatant was removed and the platelet pellet was suspended in $Ca^{2+}$-free Tyrode's buffer (containing 1 mM $Ca^{2+}$). Apyrase was added to the platelet and cultured for 15 min at 37°C, and then EDTA was added (final concentration was 5 mM) and centrifuged at 1000g for 6 min. After centrifugation, the platelet pellet was resuspended in $Ca^{2+}$- Tyrode's buffer (containing 1 mM $Ca^{2+}$, pH=7.3) and the turbidity of suspension was adjusted to 0.6 (about $2.5 \times 10^8$ platelets/ml). The turbidity was determined with a Platelet Aggregation Chromogenic Kinetic System.

**B) Determination of Platelet Aggregation**

0.5ml of platelet suspension was added to silicon-coated cuvette and put in a Platelet Aggregation Chromogenic Kinetic System and bathed for 2 min at 37°C. The essential oils of herbs were added in platelet suspension. Two min. later, PAF was added to the platelet suspension. The platelet aggregation was continually observed for 4 min. The ratio of aggregation was calculated by the following formula:

$$\text{Ratio of aggregation}\% = (\text{absorptivity before adding PAF} - \text{absorptivity after adding PAF} / (\text{absorptivity before adding PAF} - \text{absorptivity of Tyrode's buffer}) \times 100\%$$

The ratio of inhibition was calculated by the following formula:

$$\text{Ratio of inhibition }\% = 100\% - (\text{ratio of aggregation of sample} / \text{ratio of aggregation of control} \times 100\%)$$

The IC50 values are shown in below Table 2.

Table 2

| Samples | $IC_{50}$(mg/ml) |
|---|---|
| *Magnolia biondii* | 41±5.5 |
| *Agastache rugosa* | 10.5±0.1 |
| *Notopterygium forbesii* | 19.5±1.5 |
| *Magnolia biondii + Agastache rugosa* (1:1 by volume) | 9±1 |
| *Magnolia biondii + Notopterygium forbesii* (1:1 by volume) | 27±3.4 |
| *Magnolia biondii + Agastache rugosa + Notopterygium forbesii* (1:1:1 by volume) | 20±2 |
| CV-3988 | 1.42 |

Example 3: In vivo assay of Essential Oils from Herbs of the Present Invention

Fifty-six male, 8-10-week-old, Balb/c mice, purchased from the National Laboratory Animal Breeding and Research Center, Taipei, Taiwan, were used in this example. The mice were randomly divided into 8 groups (7 mice/group). Each group was treated by a different process listed as follows:

I) PBS group: The mice were sensitized by intraperitioneal injection and challenged by intranasal challenge with PBS.

II) OVA group: The mice were sensitized by intraperitioneal injection and challenged by intranasal challenge with OVA.

III) Essential oil of *Magnolia biondii* group (MB group): The mice were sensitized by intraperitioneal injection and challenged by intranasal challenge with OVA and then treated with essential oil of *Magnolia biondii.*

IV) Essential oil of *Agastche rugosa* group (AR group): The mice were sensitized by intraperitioneal injection and challenged by intranasal challenge with OVA and then treated with essential oil of *Agastche rugosa.*

V) Essential oil of *Notopterygium forbesii* group (NF group): The mice were sensitized by intraperitioneal injection and challenged by intranasal challenge with OVA and then treated with essential oil of *Notopterygium forbesii.*

VI) Essential oil of *Magnolia biondii* and *Agastche rugosa* and *Notopterygium forbesii* group (MAN group): The mice were sensitized by intraperitioneal injection and challenged by intranasal challenge with OVA and then treated with essential oil of *Magnolia biondii* and *Agastche rugosa* and *Notopterygium forbesii.*

VII) Essential oil of *Magnolia biondii* and *Agastche rugosa* group (MA group): The mice were sensitized by intraperitioneal injection and challenged by intranasal challenge with OVA and then treated with essential oil of *Magnolia biondii* and *Agastche rugosa.*

VIII) Essential oil of *Magnolia biondii* and *Notopterygium forbesii* group (MN group): The mice were sensitized by intraperitioneal injection and challenged by intranasal challenge with OVA and then treated with essential oil of *Magnolia biondii* and *Notopterygium forbesii.*

The Ovalbumin (OVA, Sigma A-5378) was used as an allergen to sensitize the mice, and aluminum potassium sulfate (Sigma A-7167) was used as adjuvant. The OVA and aluminum potassium sulfate were dissolved in a PBS buffer. The resulting solution was particularly administered to each mouse on the 1st day, 5th day, 14th day, and 21st day by intraperitioneal injection. 0.1 ml/mouse of the solution was administered.

Twenty-one days after sensitization, the mice were continually challenged with OVA solution (25mg/ml) daily, dissolved in PBS buffer, for three weeks by intranasal challenge. The dosage of administration was $20\mu$ 1/mice/day. The essential oil was vaporized in a closed and sterilized chamber with an electro-thermal hot plate, and then the case was filled with essential oil steam. Ten min. after being challenged, the mice were placed into the case for 30 min.

The clinical behaviour of mice were diagnosed on the 21st day, 28th day, 35th day, and 42nd day after the challenge.

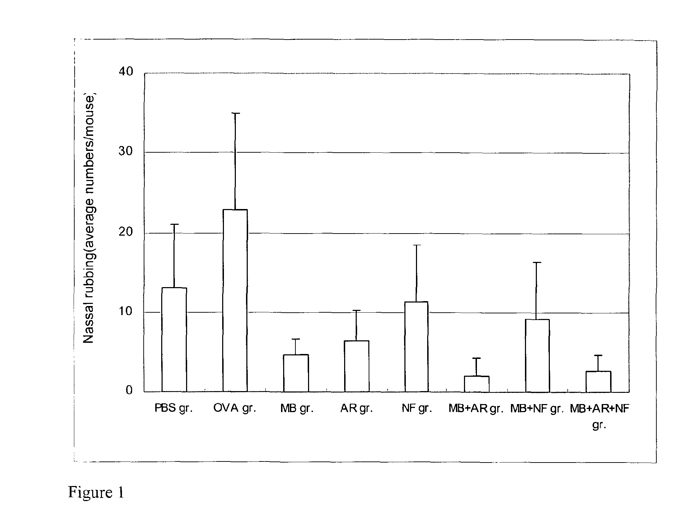

Figure 1 shows the average number of nasal rubbings counted during the 10 min. after the mice were challenged by intranasal challenge with OVA. The results show that the average number of nasal rubbings significantly decreased after the mice were treated compositions of the invention having essential oil of herbal medicine.

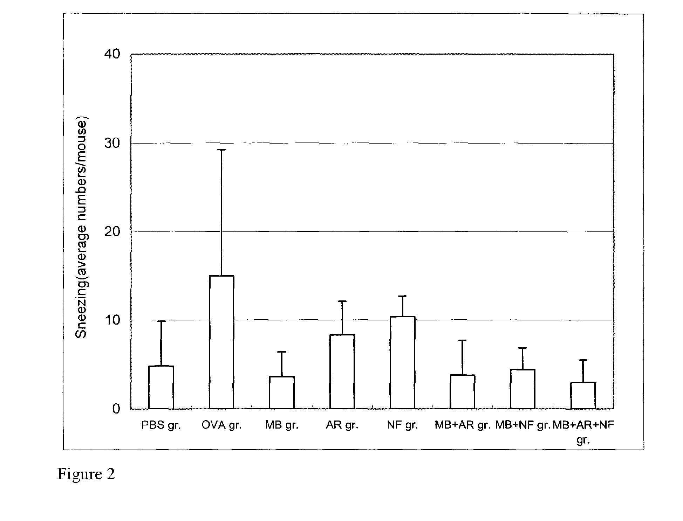

Figure 2 shows the average number of sneezes counted during the 10 min. after the mice were challenged by intranasal challenge with OVA. The results show that the average numbers of sneezing significantly decreased after the mice were treated with compositions of the invention having essential oil of herbal medicine.

**Claims**

1. A composition for the treatment and/or prevention of allergic disease, which comprises an (essential) oil of HOUXIANG HERBA and/or an (essential) oil of XINYI HERB, wherein HOUXIANG HERBA comprises the plant *Agastache rugosa, Pogostemon cablin, Pogostemon formosanum, Microtoena delavayi, Microtoena insuavis, Microtoena patchouli, Teucrium quadrifarium, Epimeredi indica* or *Anisomeles indica,* and XINYI HERB comprises the plant *Magnolia biondii, Magnolia denudate Dear., Magnolia denudate Desr, Magnolia Pilocarpa, Magnolia liliiflora, Magnolia sargentiana, Magnolia cempbelli Hook., Magnolia martini Dandy* or *Magnolia sprengeri.*

2. A composition according to Claim 1, wherein the HOUXIANG HERB is from *Agastaclae rugosa* and/or XINYI HERB is *Magnolia biondii.*

3. A composition according to Claim 1 or 2, wherein the oil of HOUXIANG HERB ranges from 0.05% to 0.95% (or parts) by volume, and/or the oil of XINYI HERB ranges from 0.05% to 0.95% (or parts) by volume in the composition.

4. A composition for the treatment and/or prevention of allergic disease, which comprises an oil of QIANGHUO HERBA and an oil of XINYI HERB (or magnosalin), wherein QIANGHUO HERBA comprises the plant *Notopterygium forbesii* or *Notopterygium incisum* and XINYI HERB comprises the plant *Magnolia biondii, Magnolia denudata Dear., Magnolia denudata Desr., Magnolia pilocarpa, Magnolia liliiflora, Magnolia sargentiana, Magnolia cempbelli Hook., Magnolia martini Dandy,* or *Magnolia sprengeri.*

5. A composition according to Claim 4, wherein the QIANGHUO HERB is *Notopterygium forbesii* and/or the XINYI HERB is *Magnolia biondii.*

6. A composition according to Claim 4 or 5, wherein said essential oil of QIANGHUO HERB in the composition ranges from 0.05% to 0.95% (or parts) by volume, and the essential oil of XINYI HERB (magnosalin) in the composition ranges from 0.05% to 0.95% (or parts) by volume.

7. A composition for the treatment and/or prevention of allergic disease, which comprises an (essential) oil of HOUXIANG HERBA, an (essential) oil of QIANGHUO HERB and/or an (essential) oil of XINYI HERB (or magnosalin), wherein HOUXIANG HERB comprises the plant *Agastache rugosa, Pogostemon cablin, Pogostemon formosanum, Microtoena delavayi, Microtoena insuavis, Microtoena patchouli, Teucrium quadrifarium, Epimeredi indica* or *Anisomeles indica;* QIANGHUO HERBA comprises the plant *Notopterygium forbesii* or *Notopterygium incisum;* and or XINYI HERB comprises the plant *Magnolia biondii, Magnolia denudata Dear., Magnolia denudata Desr., Magnolia pilocarpa, Magnolia liliiflora, Magnolia sargentiana, Magnolia cempbelli Hook., Magnolia martini Dandy* or *Magnolia sprengeri.*

8. A composition according to Claim 7, wherein the HOUXIANG HERB is *Agastache rugosa,* QIANGHUO HERB is *Notopterygium forbesii* and/or XINYI HERB is *Magnolia biondii.*

9. A composition according to Claim 7 or 8, wherein the composition comprises 0.5% to 99.5% by volume of the oil of HOUXIANG HERBA, 0.5% to 99.5% by volume of the oil of QIANGHUO HERBA and/or 0.5% to 99.5% by volume of the essential oil of XINYI HERB.

10. A composition according to any preceding Claim, wherein the allergic disease comprises allergic rhinitis, allergic pneumonia, allergic conjunctivitis, allergic dermatitis, allergic sinusitis, allergic asthma, allergic bronchitis, allergic arthritis, allergic arteritis or a food allergy.

11. A composition according to Claim 10, wherein the allergic disease comprises allergic rhinitis.

12. Use of an oil of HOUXIANG HERBA in the manufacture of a medicament for treating and/or preventing allergic disease, wherein the HOUXIANG HERBA comprises the plant *Agastache rugosa, Pogostemon cablin, Pogostemon formosanum, Microtoena delavayi, Microtoena insuavis, Microtoena patchouli, Teucrium quadrifarium, Epimeredi indica* or *Anisomeles indica.*

13. The use according to claim 12 wherein the HOUXIANG HERB comprises *Agastache rugosa.*

14. Use of an oil of QIANGHUO HERBA in the manufacture of a medicament for treating and/or preventing allergic disease, wherein the QIANGHUO HERBA comprises the plant *Notopterygium forbesii* or *Notopterygium incisum.*

15. The use according to Claim 14, wherein the QIANGHUO HERB comprises *Notopterygium forbesii.*

16. The use of a composition, or a component oil of thereof, according to any of claims 1 to 12 in the manufacture of a medicament for treating and/or preventing allergic disease.

17. The use according to any of Claim 12 to 15, wherein the allergic disease comprises includes allergic rhinitis, allergic dermatitis, allergic pneumonia, allergic conjunctivitis, allergic dermatitis, allergic sinusitis, allergic asthma, allergic bronchitis, allergic arthritis, and allergic arteritis and food allergies.

18. The use according to Claim 16, wherein the allergic disease comprises allergic rhinitis.

19. The use according to any of Claims 12 to 16, wherein the oil is adapted to be administered to a patient by inhalation delivery, oral delivery, intranasal delivery, transdermal delivery, percutaneous absorption and/or a hot bath.

Figure 1

Figure 2

European Patent Office

**EUROPEAN SEARCH REPORT**

Application Number

EP 05 25 4158

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | ANONYMOUS: "AllerAid Herbal (Hypoallergenic)" INTERNET ARTICLE, [Online] April 2005 (2005-04), XP002341142 Retrieved from the Internet: URL:http://www.allergyresearchgroup.com/proddesc/discuss/AllerAidHerbalPDFProductSheet040105.pdf> [retrieved on 2005-08-16] * page 2, paragraph 2 * | 1-3, 7-13, 16-19 | A61K35/78 A61P37/08 |
| X | DATABASE WPI Section Ch, Week 199731 Derwent Publications Ltd., London, GB; Class B03, AN 1997-333335 XP002341358 & CN 1 106 304 A (WANG J) 9 August 1995 (1995-08-09) * abstract * | 1-3, 7-13, 16-19 | |
| X | DATABASE WPI Section Ch, Week 199637 Derwent Publications Ltd., London, GB; Class B04, AN 1996-368115 XP002341359 & JP 08 176002 A (KAO CORP) 9 July 1996 (1996-07-09) * abstract * | 1-3, 7-13, 16-19 | TECHNICAL FIELDS SEARCHED (Int.Cl.7) A61K A61P |
| X,P | OH HWA MIN ET AL: "Agastache rugosa leaf extract inhibits the iNOS expression in ROS 17/2.8 cells activated with TNF-alpha and IL-1 beta" ARCHIVES OF PHARMACAL RESEARCH (SEOUL), vol. 28, no. 3, March 2005 (2005-03), pages 305-310, XP009052463 ISSN: 0253-6269 * abstract * | 1-3, 7-13, 16-19 | |

-/--

The present search report has been drawn up for all claims

| Place of search | Date of completion of the search | Examiner |
|---|---|---|
| Munich | 19 August 2005 | Büttner, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 632 240 A1**

European Patent Office

**EUROPEAN SEARCH REPORT**

Application Number

EP 05 25 4158

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE WPI<br>Section Ch, Week 200260<br>Derwent Publications Ltd., London, GB;<br>Class B04, AN 2002-563544<br>XP002341360<br>& KR 2002 013 134 A (PARK C W)<br>20 February 2002 (2002-02-20)<br>* abstract * | 1-3,<br>7-13,<br>16-19 | |
| X | WONG H C G: "Chinese herbal medicine and allergy"<br>ALLERGY AND CLINICAL IMMUNOLOGY INTERNATIONAL, HANS HUBER PUBLICATIONS, TORONTO, CA,<br>vol. 13, no. 5, 2001, pages 192-196,<br>XP002956614<br>ISSN: 0838-1925<br>* page 193; table 1 * | 1-3,<br>7-13,<br>16-19 | |
| X | KATAOKA MASAHIRO ET AL: "Effect of the crude drugs (standards of natural drugs not in the J.P. XII) on beta-hexosaminidase release from rat basophilic leukemia (RBL-2H3) cells"<br>NATURAL MEDICINES,<br>vol. 49, no. 3, 1995, pages 346-349,<br>XP009052464<br>ISSN: 1340-3443<br>* page 349; table 1 * | 1-3,<br>7-13,<br>16-19 | TECHNICAL FIELDS SEARCHED (Int.Cl.7) |
| X,P | LI J ET AL: "Lignan and neolignan derivatives from Magnolia denudata"<br>CHEMICAL AND PHARMACEUTICAL BULLETIN 2005 JAPAN,<br>vol. 53, no. 2, 2005, pages 235-237,<br>XP002341143<br>ISSN: 0009-2363<br>* abstract *<br>-----<br>-/-- | 1-11,<br>16-19 | |

The present search report has been drawn up for all claims

| Place of search | Date of completion of the search | Examiner |
|---|---|---|
| Munich | 19 August 2005 | Büttner, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

**EUROPEAN SEARCH REPORT**

Application Number

EP 05 25 4158

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | KIM H M ET AL: "Shini-San inhibits mast cell-dependent immediate-type allergic reactions" AMERICAN JOURNAL OF CHINESE MEDICINE, vol. 27, no. 3-4, 1999, pages 377-386, XP009052530 ISSN: 0192-415X * page 378, last paragraph * * page 384, last paragraph * ----- | 1-11, 16-19 | |
| X | KIM GYOO C ET AL: "Magnoliae flos induces apoptosis of RBL-2H3 cells via mitochondria and caspase." INTERNATIONAL ARCHIVES OF ALLERGY AND IMMUNOLOGY, vol. 131, no. 2, June 2003 (2003-06), pages 101-110, XP009052532 ISSN: 1018-2438 * page 102, column 1 * * page 107, column 2, paragraph 1 * ----- | 1-11, 16-19 | |
| X | MATSUDA HISASHI ET AL: "Effects of constituents from the bark of Magnolia obovata on nitric oxide production in lipopolysaccharide-activated macrophages" CHEMICAL AND PHARMACEUTICAL BULLETIN (TOKYO), vol. 49, no. 6, June 2001 (2001-06), pages 716-720, XP001207307 ISSN: 0009-2363 * page 718; table 1 * * page 718, column 2 * ----- -/-- | 1-11, 16-19 | **TECHNICAL FIELDS SEARCHED** (Int.Cl.7) |

The present search report has been drawn up for all claims

| Place of search | Date of completion of the search | Examiner |
|---|---|---|
| Munich | 19 August 2005 | Büttner, U |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    .....................................................................................
& : member of the same patent family, corresponding
    document

EPO FORM 1503 03.82 (P04C01)

**European Patent Office**

**EUROPEAN SEARCH REPORT**

Application Number

EP 05 25 4158

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | ACHIKE FRANCIS I ET AL: "Nitric oxide, human diseases and the herbal products that affect the nitric oxide signalling pathway."<br>CLINICAL AND EXPERIMENTAL PHARMACOLOGY AND PHYSIOLOGY,<br>vol. 30, no. 9, September 2003 (2003-09), pages 605-615, XP002341144<br>ISSN: 0305-1870<br>* page 607, column 2, paragraph 3 *<br>* page 611; table 2 *<br>----- | 1-19 | |
| X | US 4 537 996 A (KOODA ET AL)<br>27 August 1985 (1985-08-27)<br>* column 6, line 18 - line 45 *<br>----- | 1-11, 16-19 | |
| X | DATABASE WPI<br>Section Ch, Week 200425<br>Derwent Publications Ltd., London, GB;<br>Class B04, AN 2004-264209<br>XP002341361<br>& KR 2003 091 220 A (HWANG C Y)<br>3 December 2003 (2003-12-03)<br>* abstract *<br>----- | 1-11, 16-19 | |
| X | DATABASE WPI<br>Section Ch, Week 200169<br>Derwent Publications Ltd., London, GB;<br>Class B04, AN 2001-605341<br>XP002341362<br>& JP 2001 192338 A (POLA CHEM IND INC)<br>17 July 2001 (2001-07-17)<br>* abstract *<br>-----<br>-/-- | 4-11, 14-19 | |

TECHNICAL FIELDS SEARCHED (Int.Cl.7)

The present search report has been drawn up for all claims

| Place of search | Date of completion of the search | Examiner |
|---|---|---|
| Munich | 19 August 2005 | Büttner, U |

EPO FORM 1503 03.82 (P04C01)

**EP 1 632 240 A1**

European Patent
Office

**EUROPEAN SEARCH REPORT**

Application Number

EP 05 25 4158

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE WPI<br>Section Ch, Week 200463<br>Derwent Publications Ltd., London, GB;<br>Class B04, AN 2004-643121<br>XP002341363<br>& CN 1 507 894 A (SCI & TECH IND PARK CO LTD GUANGZHOU TRA)<br>30 June 2004 (2004-06-30)<br>* abstract *<br>----- | 1-3 | |
| X | DATABASE WPI<br>Section Ch, Week 200241<br>Derwent Publications Ltd., London, GB;<br>Class B04, AN 2002-372677<br>XP002341364<br>& CN 1 179 965 A (LI C)<br>29 April 1998 (1998-04-29)<br>* abstract *<br>----- | 1-3,7-11 | |
| | | | TECHNICAL FIELDS SEARCHED (Int.Cl.7) |

The present search report has been drawn up for all claims

| Place of search | Date of completion of the search | Examiner |
|---|---|---|
| Munich | 19 August 2005 | Büttner, U |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 05 25 4158

This annex lists the patent family members relating to the patent documents cited in the above-mentioned European search report.
The members are as contained in the European Patent Office EDP file on
The European Patent Office is in no way liable for these particulars which are merely given for the purpose of information.

19-08-2005

| Patent document cited in search report | | Publication date | Patent family member(s) | | Publication date |
|---|---|---|---|---|---|
| CN 1106304 | A | 09-08-1995 | NONE | | |
| JP 8176002 | A | 09-07-1996 | NONE | | |
| KR 2002013134 | A | 20-02-2002 | NONE | | |
| US 4537996 | A | 27-08-1985 | JP | 1776003 C | 28-07-1993 |
| | | | JP | 4063054 B | 08-10-1992 |
| | | | JP | 59095229 A | 01-06-1984 |
| | | | AU | 567509 B2 | 26-11-1987 |
| | | | AU | 1927483 A | 31-05-1984 |
| | | | US | 4650813 A | 17-03-1987 |
| KR 2003091220 | A | 03-12-2003 | NONE | | |
| JP 2001192338 | A | 17-07-2001 | NONE | | |
| CN 1507894 | A | 30-06-2004 | NONE | | |
| CN 1179965 | A | 29-04-1998 | NONE | | |

EPO FORM P0459

For more details about this annex : see Official Journal of the European Patent Office, No. 12/82